Europäisches Patentamt

European Patent Office

Office européen des brevets

(19)

(11) Publication number: **0 241 444**
**A1**

(12) **EUROPEAN PATENT APPLICATION**

(21) Application number: **87850117.0**

(22) Date of filing: **08.04.87**

(51) Int. Cl.³: **G 01 N 33/531**
**A 61 K 39/395, C 07 K 15/00**
**G 01 N 33/577, G 01 N 33/5-**
**66**

(30) Priority: **11.04.86 SE 8601645**

(43) Date of publication of application:
**14.10.87 Bulletin 87/42**

(84) Designated Contracting States:
**AT BE CH DE ES FR GB GR IT LI LU NL SE**

(71) Applicant: **Nilsson, Ulf Ragnar**
**Storgatan 18 C**
**S-753 31 Uppsala(SE)**

(71) Applicant: **Nilsson, Sven Ivar Bo**
**Byggmästargatan 10D**
**S-754 35 Uppsala(SE)**

(71) Applicant: **Svensson, Karl-Erik**
**Västertorg 5A**
**S-752 43 Uppsala(SE)**

(72) Inventor: **Nilsson, Ulf Ragnar**
**Storgatan 18 C**
**S-753 31 Uppsala(SE)**

(72) Inventor: **Nilsson, Sven Ivar Bo**
**Byggmästargatan 10D**
**S-754 35 Uppsala(SE)**

(72) Inventor: **Svensson, Karl-Erik**
**Västertorg 5A**
**S-752 43 Uppsala(SE)**

(74) Representative: **Widén, Björn et al,**
**Uppsala Patentbyra Box 9039**
**S-750 09 Uppsala(SE)**

(54) **Immunological method involving formation of ]anti-receptor:anti-ligand[ antibody complex.**

(57) In a immunological method antibodies are produced which are capable of reacting with a specific part of a contact surface of either of two macromolecules interacting to form a complex, the method comprising

a) separately producing polyclonal antisera containing antibodies against the respective macromolecules,

b) contacting the immunoglobulin fractions of said antisera with each other, such that complementary antibodies may react to form antibody complexes,

c) separating the pairs of complexed antibodies obtained from antibodies which have not reacted with each other, and

d) dissociating said antibody complexes and separately recovering at least one antibody component of said antibody complexes.

The antibodies obtained may be used for identifying and recovering antigens or fragments thereof from a mixture or as substitute antigens.

FIG. 1B

FIG. 1C

Immunological method

The present invention relates to the production of anti-idiotype antibodies and more particularly it relates to a method for the production of antibodies directed against complementary binding domains of complexing macromolecules as well as the use thereof, i.a. for identifying and isolating such binding domains.

Interacting biological macromolecules which form complexes with each other are bound via special substructures which are sterically complementary to each other. The determination of such functional domains of, e.g., proteins and cell surface structures has hitherto been a heavy and time-consuming work. In addition to its value in pure basic research the identification of such domains has importance in, for example, the production of vaccines, drugs, etc. Until now both polyclonal and monoclonal antibody reagents have been used in these determinations. Principally such a determination has been effected by first fragmenting the protein in question and isolating and characterizing the fragments obtained. Then specific antisera against the fragments have been produced, whereupon a possible association between function and a certain sub-fragment of the protein has been demonstrated by binding of the respective specific antibodies to the protein.

In addition to being laborious the method has a number of obvious disadvantages. Thus, for example, the resolving capacity of the method depends i.a. on how large and well-defined the studied fragment is. Further, as is well-known, a protein fragment will lose much of its antigenic properties when broken down into smaller units. A further disadvantage, at least as far as the use of polyclonal antibody reagents is concerned, is that the possibility of providing stereospecific anti-"site" antibodies is extremely restricted, not to say non-existent. When using polyclonal reagents it may further not be taken for granted that the inhibition of the function of a macromolecule depends on blocking of an active or binding site, but it may also depend on other effects, such as, e.g., insolubilization by immunoprecipitation, that antibodies bound to other sites than the active domain constitute pure mechanical hinders or effect the conformation of the active molecule in a disadvantageous way, etc.

Even if the last-mentioned disadvantages may be reduced or eliminated to a great extent by the use of monoclonal antibodies, a rather extensive preparatory work in the form of screening of a great number of immunoglobulin producing clones is required before suitable reagents for the study of function-related active sites of interest are obtained.

According to the present invention a method is suggested by which a great deal of work in the hitherto used methodologies is avoided and the structures involved in the binding between two interacting macromolecules may be identified relatively directly and efficiently distinguished from other structures located outside the actual contact surfaces. By the method of the invention antigenic part structures within the binding surfaces may be identified as well as antibodies against them be produced.

The invention is based upon the concept that in order to identify the specific domains or surfaces of a pair of interacting macromolecules, e.g., protein-cell receptor, which are directly involved in the binding, one utilizes the fact that antisera against the two macromolecules each contain certain antibodies directed against the binding domain of the respective macromolecule, which two groups of antibodies therefore should, on one hand, exhibit anti-idiotype specificity to each other (i.e. have complementary binding structures) and, on the other hand, immunologically correspond to the binding domain of the respective macromolecule, and that one selects these binding-specific antibodies by mixing the two antibody preparations such that an isolatable complex is formed between these anti-idiotype antibodies, and subsequently dissociating the complexes into individual antibodies. In such manner antibodies specifically directed against a binding domain, which is not known in any detail, of a macromolecule may readily be produced, and the antibodies may then be utilized for isolating the antigen fragment in question.

One aspect of the present invention thus relates to a method for the production of antibodies capable of reacting with a specific part of a contact surface of either of two macromolecules interacting to form a complex, said method comprising

a) separately producing polyclonal antisera containing antibodies against the respective macromolecules,

b) contacting the immunoglobulin fractions of said antisera with each other such that complementary antibodies may react to form antibody complexes,

c) separating the pairs of complexed antibodies obtained from non-reacted antibodies, and

d) dissociating said antibody complexes and separately recovering at least one antibody component of said antibody complex.

According to a further aspect of the present invention the antibodies obtained in step d) above are utilized to identify and recover an antigen or a fragment thereof taking part in the above mentioned macromoleculear interaction from a mixture containing the corresponding macromolecule or fragment thereof, respectively.

In many cases it may be advantageous to before or after step b) subject one or both antisera to an adsorptive purification step for removing non-specifically reacting immunoglobulins. Such adsorption may, for example, be performed against normal rabbit Ig or pre-immune Ig.

The two above mentioned interacting macromolecules may be selected from a variety of interacting pairs and may, for example, be an enzyme and its substrate, an enzyme and its inhibitor, a hormone and its cell receptor, two interacting complement factors, etc., or considerably more complex structures, such as, for example, a whole cell and a parasite, or an infectious agent binding to the cell surface. Examples of pairs of the last-mentioned type are cell-virus, cell-bacteriae, cell-protozoo. Principally the pair of macromolecules may also be an antibody and its homologous antigen, but as is readily appreciated such application would not be of any practical interest. According to a more restricted aspect of the invention said macromolecular pair is therefore not antigen-antibody.

Hereinafter the invention will be explained in more detail with reference to the accompanying drawings, wherein

Fig. 1A diagrammatically illustrates two interacting macromolecules;

Fig. 1B illustrates the respective macromolecules together with antibodies against the substructures thereof;

Fig. 1C shows the result of combination of the antibodies of the two macromolecules; and

Fig. 2 diagrammatically illustrates the structure of the complement factor C3 and its cleavage.

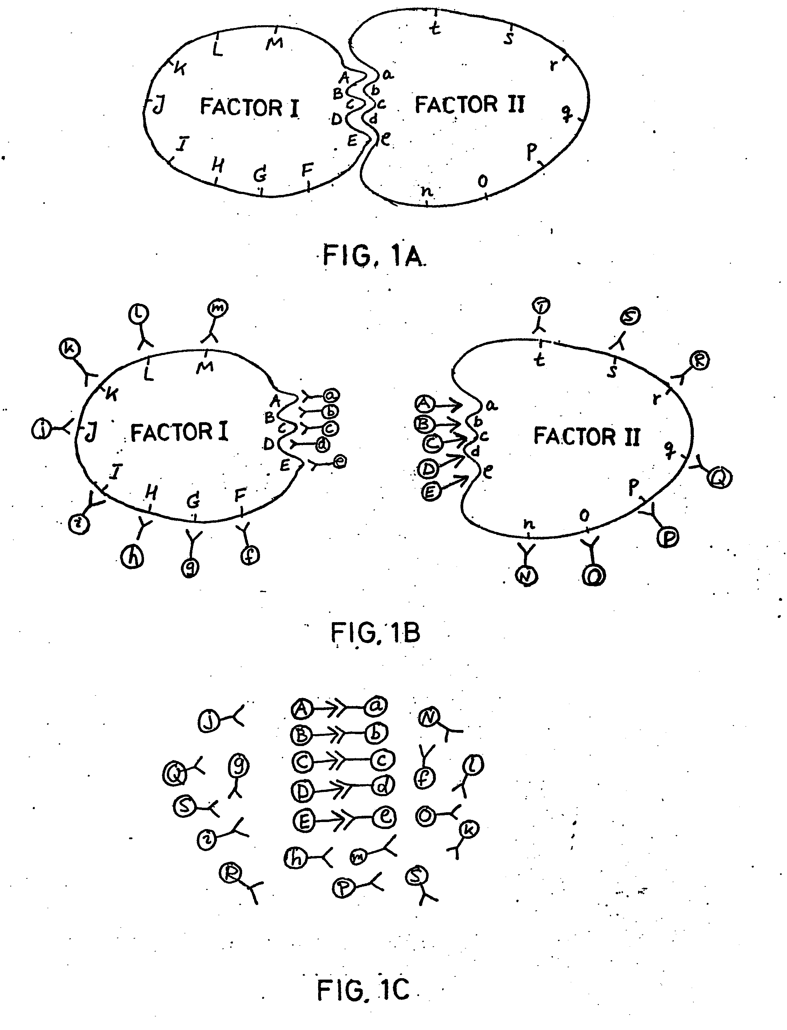

In Figure 1A the above mentioned interacting macromolecules are each represented by a respective block designated as factor I and factor II, respectively. These factors may enter into a binding reaction via complementary surfaces or substructures designated as A-E for factor I and $\underline{a}$-$\underline{e}$ for factor II, the substructure A being complementary to the substructure $\underline{a}$, etc. If now polyclonal antisera against the respective factors I and II are produced according to step a) of the above defined method, the antiserum against factor I will contain antibodies against each substructure A, B, C, etc., symbolized by ⓐ─< , ⓑ─< , ⓒ─< , etc. in Fig. 1B, while the antiserum against factor II will contain antibodies against the substructures $\underline{a}$, $\underline{b}$, $\underline{c}$, etc. and symbolized by Ⓐ─>, Ⓑ─>, Ⓒ─>, etc. in Fig. 1B. Of the antibodies formed against the respective factor only a limited number thereof are directed against the common contact surfaces of the factors I and II and thereby complementary to each other, viz., on one hand, the antibodies against the substructures A-E, and, on the other hand, the antibodies against the substructures $\underline{a}$-$\underline{e}$. Most of the antibodies, i.e. those against the substructures F-M and $\underline{n}$-$\underline{t}$, respectively, are,

4

however, not complementary to each other. Thus, in their specificity the antibodies against A-E and the antibodies against a-e are a set of anti-idiotype antibody pairs. It is to be noted that it is not necessary for the two macromolecules I and II to be produced in pure form, but they may each individually be included in a mixture containing, e.g., other macromolecules.

If now according to step b) of the above defined method the antiserum against factor I obtained is mixed with the antiserum against factor II, the anti-idiotype antibodies are bound to each other, while the other antibodies will remain in their free form as is shown in Fig. 1C. This complexing is selective for antibodies against the complementary binding surfaces of factors I and II and may therefore be used to isolate these antibodies. This may, for example, be performed by prior to the mixing of the above mentioned antisera binding the immunoglobulin fraction of one antiserum (say the immunoglobulins directed against factor I) to a solid phase and then carrying out step b) of the method by contacting the other antiserum (i.e. in this case the anti-factor II preparation) with the solid phase bound immunoglobulins. The antibodies which have not been bound to the solid phase are then separated off in accordance with step c) of the method, whereupon, in accordance with method step d) the anti-idiotype antibodies (against factor II) adsorbed to the solid phase are dissociated from the idiotype antibodies (against factor I) initially bound to the solid phase and the dissociated antibodies are recovered.

The above mentioned isolation of the antibodies (method steps c and d) may advantageously be carried out by affinity chromatografic means, i.e., by coupling one antibody preparation to a suitable carrier, e.g., Sepharose® (Pharmacia AB), packed in a column, and then passing the other antiserum through the column. Hereby, as above, anti-idiotype antibodies will bind to the column, while other antibodies will be washed away. Then the anti-idiotype antibodies may in per se known manner be eluted by an acid buffer.

Such isolation of the antibodies may, of course, also be effected in other ways, e.g., by mixing the antisera obtained, precipitating the complexes by changing the pH and/or ion strength, and dissociating and recovering the desired antibodies in any suitable manner.

The antibodies obtained as above are in the specific circumstances indicated of anti-factor II type with specificity against the substructures a-e, and are symbolized by Ⓐ→ to Ⓔ→ in Fig. 1B. These antibodies thus have on their antigen-binding structures configurations which are identical with binding structures of factor I. In analogous manner one may, of course, instead isolate the binding specific antibodies against factor I, which thus have

5

configurations of their antigen-binding structures identical with binding structures of factor II. It is to be noted, this being an important feature of the invention, that, for example, the anti-idiotype anti-A (ⓐ—ᐸ ) has not been produced in conventional manner against its idiotype anti-body, i.e. anti-a (Ⓐ—→ ), but against an antigen A which is complementary to the antigen a.

The antibodies obtained may, optionally after further purification, be used as substitute antigens for the antigen structures to which they correspond in accordance with the above discussion, and such use will be described in more detail further on. Alternatively, according to the other aspect of the invention, the antibodies obtained may be used to identify and select specific antigenic structures, such as, e.g., within the factor II binding surface against factor I, or vice versa, as will be described in more detail hereinafter.

To identify antigens within the factor II binding surface against factor I, factor II is broken down or degraded in conventional manner into antigen fragments, e.g., by reduction, enzymatic or chemical cleavage, etc.. If, for example, factor II is a protein, it may be dissociated down into its polypeptide chains in a reducing-dissociating medium. Further degradation may be effected by proteolysis in the presence of selected proteolytic enzymes. As a result of these degradation procedures a complex mixture will be obtained, in which the structures forming the binding sites of the corresponding intact factor or macromolecule are to be identified. The complex antigen solutions obtained may then be analysed with anti-II antibodies obtained, e.g., by affinity purification as above with insolubilized anti-I immunoglobulins. These antibodies will then react with antigens located in the factor II binding surface against factor I. To visualize the resulting reaction obtained between the antigen sought-after and the binding site specific antibodies any suitable conventional technique may be used.

For analytical scale work, for example, so-called immunoblotting technique may be used. In this technique the antigen mixture is separated by electrophoresis, isoelectric focusing or in any other suitable manner, whereupon the antigens are transferred onto a nitrocellulose membrane. This transfer may be effected by electrophoresis or by direct contact under pressure. The nitrocellulose membrane is then incubated with an excess of protein to block free binding sites, after which the affinity-purified antibodies obtained above are added. To detect the antibodies bound to the membrane enzyme-labelled anti-antibody against the these antibodies is then added followed by an enzyme substrate, or alternatively radioactively labelled anti-antibody.

The above mentioned immunoblotting technique may also be used for preparative scale separation, although ELISA (enzyme linked immunosorbent assay) technique might be preferable in this case. For example, microtitre plates may be coated with material from the different antigen fractions and the kind of antigen adsorbed may then be analysed by means of the affinity-purified antibodies by ELISA technique, i.e. labelling the bound antibodies by means of enzyme anti-antibody conjugate (e.g., alkaline phosphatase, peroxidase, β-galactosidase or the like), and then measuring the enzyme activity, e.g., colorimetrically or fluorimetrically, upon addition of substrate.

In the above described manner the antigenic structures in, e.g., the factor II binding surface against factor I may be isolated. The complexity of the antigen mixture resulting after the degradation procedure will, of course, increase according to the complexity of the macromolecule and will, for example, be significant when, e.g., a whole cell has been degraded. It will be understood that by means of the process according to the invention for identifying the specific binding structures a considerable saving of time will be obtained in relation to the previously used methods.

The antigen component or components isolated in such manner may be utilized in various ways. For example, structure-function relations on the basic scientific plane may be investigated. Thus, for example, a submolecular structure involved in a binding reaction of a protein or a cell receptor may be identified and characterized. Further, the isolated component may also be used for screening monoclonal antibodies with specificity against a binding site of a macromolecule, e.g., in the above illustration the factor II binding site for factor I. In this case the isolated antigen fraction may be attached to a microtitre plate, with which monoclonal test antibodies are then analysed by, for example, the above mentioned ELISA technique.

The isolated antigen may also be used for producing vaccines or antisera for a passive immune protection against bacteriological, virological and parasitic diseases, such as malaria, viral and bacterial infections, etc. Thus, as an example of such pathogenetic host-parasite interactions factor I in Fig. 1A may be considered to be a host cell and factor II a parasite, bacterium, virus, etc., which binds to the host cell via a specific receptor, or vice versa. By means of the antigen or antigens isolated from such a factor pair according to the invention it is, e.g., possible to define proteins which are critical to the infectivity of the germ and its mechanism of attacking the host organism. After such proteins have been identified there is the opportunity of large scale purification or possibly production by genetic engineering. The antigen in question may then be used in vaccines against the germ of interest to build up an active immune protection in the individual. Alternatively, it may be used for the production of antisera or monoclonal antibodies, which may then be used for passive immune protection against the parasite.

As mentioned previously the antibodies obtained which correspond to the contact surfaces may also be used directly as substitute antigens for various purposes. This may, for example, be of value if the antigen in question is unstable. Thus, e.g., stable antigen kits may be provided, for instance, for industrial use. Referring again to Figure 1, for example, anti-factor II antibodies may be utilized as substitute antigens instead of factor I when screening for binding site specific antibodies against factor I, as these anti-factor II antibodies are structurally equivalent to the binding site of factor I.

A possible method for such screening may consist in first immunizing mice against factor I, harvesting antibody producing cells, and forming monoclonal antibodies by means of now conventional hybridoma technique by fusing the spleen cells with a drug resistant mouse myeloma cell line and cloning. The cell supernatants are then tested with ELISA technique by applying them to microtitre plates, the wells of which have been coated with said binding site specific anti-factor II antibodies. After washing and treatment with enzyme-conjugated anti-mouse Ig-antibodies, addition of substrate will then produce a colouring of the wells containing antibodies of the desired specificity.

An increased sensitivity in the above described analysis could probably be obtained by modifying the method to first coat the microtitre plates with anti-mouse Ig instead of anti-binding site antibodies and then add the monoclonal test antibodies from the cell cultures. After washing and addition of binding site specific anti-factor II antibodies the possible binding of the latter may be detected by ELISA technique.

Another possible application of the method of the invention is for the production of diagnostic antibodies for use in diagnostic tests for immunity against parasites, virus and bacteriae. A diagnostic test for, e.g., anti-parasite antibodies could, of course, directly use parasite-antigen. Disadvantages of this approach is, however, that the parasite antigen in question may be unstable or infective or poor in relevant antigens for determination of immunity. According to the present invention binding site specific anti-host cell antibodies for use in the diagnostic test instead of parasite antigen are produced. Such a reagent would be particularly sensible to antibodies relevant for the immunity in question. With reference to the production system shown in Fig. 1 factor I may be considered to correspond to the parasite and factor II to the host cells, and binding site specific antibodies may then be produced analogously as described above.

For the case that the primary polyclonal antibody preparation produced according to the invention would be difficult to prepare in a sufficient amount and with well standardized qualities, more appropriate monoclonal reagents may be developed in accordance with the above mentioned methods starting from the polyclonal reagents in question.

Hereinafter the invention will be illustrated in more detail by means of some specific, non-limiting Examples.

### EXAMPLE 1

In this Example the method of the invention is shown applied to the complex formed by the complement factor C3 of the human complement system, and a control protein called factor H, and more particularly to isolate antibodies which are specifically directed against the C3 molecule binding surface for factor H and to determine in which part of the molecule this binding surface is located.

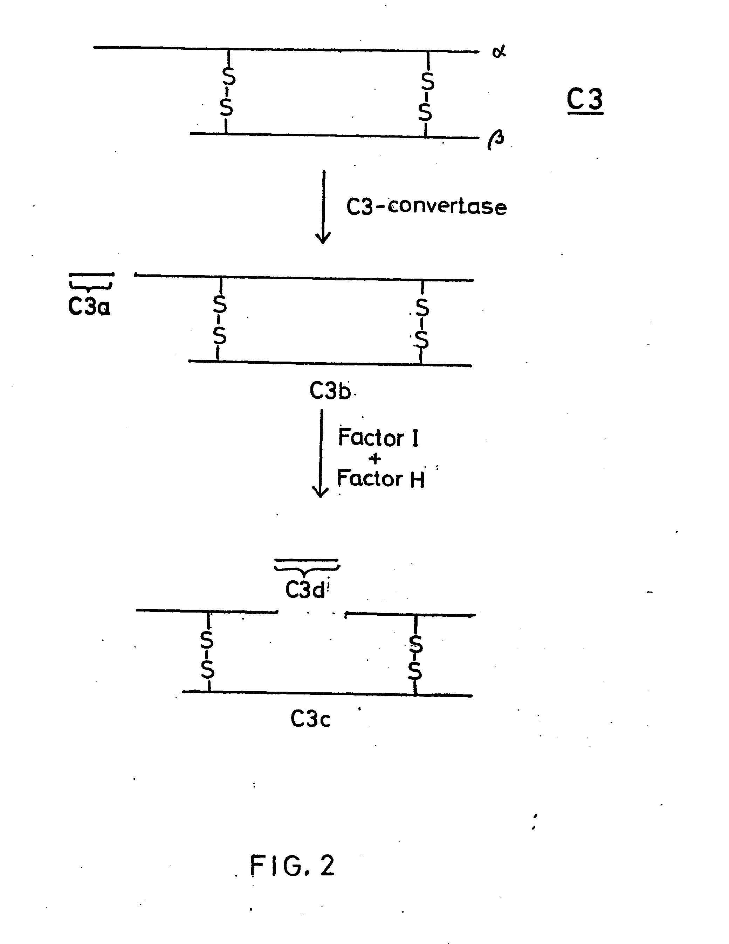

The factors C3 and H are both immunoglobulins having a molecular weight of 185 kd and 150 kd, respectively. Factor H is a markedly rod-shaped protein, while factor C3 has a more globular shape. With reference to Fig. 2 of the drawings factor C3 is composed of two polypeptide chains, designated α and β, which are kept together by disulfide bridges. The enzyme C3 convertase cleaves off a part of the α-chain of the molecule, such that fragments designated C3a and C3b are formed. A plasma enzyme, factor I (the letter) (also called C3b-inactivator) cleaves the C3b-fragment further into two part fragments, designated C3c and C3d, in the presence of factor H, which is a co-factor to factor I. The C3c-fragment consists of parts of the α-chain of the C3-molecule and the whole β-chain, while the fragment C3d is a part of the α-chain. The binding of factor H to C3 is strong, and attempts to localize the binding sites for factor H on the C3 molecule have indicated that the major binding sites would be located in the C3c portion of the molecule.

Native C3 exposes two part or subantigens, C3(S) and C3(N), which are detected by antisera produced against the native protein, designated anti-C3(SN). Denaturated C3 (e.g., after treatment with sodium lauryl sulphate (SDS)) also exposes C3(S) but no C3(N)-antigen. In this form of C3, however, C3(D)-antigens are found which have not been detected by anti-C3(SN) but well by antisera produced against the isolated, denaturated and reduced polypeptide chains α and β from C3. These antisera are called anti-C3(D) antisera.

Hereinafter the production of antibodies specific to the C3 binding domain for H will first be described. Then experiments performed with these antibodies will be described, on one hand, to show that the antibodies obtained are selected, binding domain specific antibodies to the C3-molecule and, on the other hand, to localize the binding domain of the latter.

1. Isolation of factor C3 and factor H

Human factor C3 and factor H were isolated by the method described by Nilsson, U.R., et al, Journal of Immunology 114:815-822 (1975).

2. Production of antisera against factor C3 and factor H

The factors C3 and H, respectively, isolated in step 1 above were used to immunize rabbits using the method described by Nilsson, U.R., et al, Molecular Immunology, 17:1319-1333 (1980). For factor C3 antisera were produced against native as well as denatured and reduced C3, i.e. anti-C3(SN) and anti-C3(D).

3. Coupling of anti-human factor H antibodies to the solid phase of a chromatographic column

70 mg of antibodies against human factor H, i.e. anti-human factor H antibodies, were isolated from the above obtained sera against factor H and coupled to CNBr-Sepharose® (Pharmacia AB) in the manner recommended by the manufacturer. The gel obtained was then packed in a column.

In the corresponding way another column was prepared where 70 mg of IgG isolated from normal rabbit serum had been coupled to 7 ml of CNBr-Sepharose®.

4. Affinity chromatography of anti-human factor C3

20 mg of antibodies against native human factor C3, called anti-human factor C3 antibodies, were isolated from the rabbit serum obtained in step 2 above and added to 50 ml of 0.05 M sodium phosphate containing 0.5 M sodium chloride and 0.1% (v/v) Tween® 20 (hereinafter called PB-Tween®). The resulting solution was then run through the anti-human factor H-coupled column, obtained in step 3 above, overnight at room temperature. The column was then washed with 30 ml of PB-Tween® and eluted with 0.2 M glycine containing 0.5 M sodium chloride and 0.1% Tween® 20, pH 2.8. The eluate was then dialyzed against 0.05 M sodium phosphate containing 0.15 M sodium chloride, pH 7.4 (hereinafter called PBS). This eluate, which according to the invention is to contain binding domain specific anti-human factor C3 antibodies, is for the sake of simplicity hereinafter called "E".

The above described experiment was repeated with the same antibody preparation as above, but with the second column with rabbit-IgG conjugated Sepharose® prepared in step 3. The eluate obtained was used as a control and is hereinafter designated "C".

Two additional experiments employing anti-native C3 and anti-C3α,β antibodies, respectively, were performed with two other anti-human factor H columns and the eluates were used in step 7 below.

The two eluates E and C obtained were diluted to equal volumes. To show that specific binding of anti-C3 antibodies to the anti-factor H column really had been obtained the anti-C3 IgG concentrations of the eluates E and C were compared by means of ELISA technique according to section 5 below.

5. Determination of anti-C3 activity

Constant amounts of the C3-fragment C3b in PBS containing 0.1% Tween® 20 and 0.1% (w/v) bovine serum albumin (BSA) were adsorbed to the bottom of wells of a plastic microtitre plate in a volume of 200 μl of phosphate buffered salt solution (PBS) overnight at +4°C, followed by rinsing three times with PBS-0.1% Tween® 20. To control wells only 200 μl of PBS were added.

Serial dilutions were then prepared of the two antibody eluates E and C with PBS containing 0.1% Tween® 20 and 0.1% (w/v) BSA, and 100 μl of each dilution were added to the wells of the pre-prepared microtitre plate, followed by incubation for 60 minutes at room temperature to ensure sufficient binding of the antibodies to the C3b-fragments. After rinsing three times with PBS-0.1% Tween® 20, 100 μl of swine anti-rabbit IgG conjugated with horseradish peroxidase (HRP) (DAKO Immunoglobulins A/S, Denmark) were added to bind to the anti-C3 antibodies, the HRP-conjugated antibodies being in excess of the anti-C3 antibodies. After rinsing with the same buffer as above the enzyme reaction was started by adding as a substrate to each well 100 μl of a colour reagent consisting of 20 μg of 1,2-phenylendiamine-dihydrochloride (Fluka AB, Switzerland) and 10 μl of 30% hydrogen peroxide in 75 ml of 0.1 M citrate/-phosphate buffer, pH 5.0. The reaction was stopped after approximately 10 minutes by adding 100 μl of 1 M sulphuric acid, and the absorbance (OD) at 492 nm was then measured colorimetrically.

It was found that the eluate E from the column with conjugated anti-human factor H contained about 10 times more anti-C3 antibodies than the control eluate C from the IgG column. This demonstrates that the anti-C3 antibodies were not non-specifically bound to the anti-factor H column.

The above described process was repeated, the anti-C3 antibody solution prepared in step 4 above prior to the affinity purification (hereinafter for the sake of simplicity called "O") being compared with the eluate E. As a result the binding curves obtained (absorbance versus antibody dilution) for O and E were found to have the same height and slope. The ELISA method described was then used to adjust the original antibody solution O to the same concentration as that of the eluate E. The resulting solutions were then used in the experiments 6 and 7 below to demonstrate the binding specificities of the respective antibody preparations.

6. **Comparison of binding specificity of original antibodies (O) and affinity purified antibodies (E)**

The specificities of the original antibodies O, the affinity purified antibodies E and the control eluate C with regard to the C3-fragments C3c, C3d, and isolated $\alpha$- and $\beta$-chains, respectively, were determined by repeating the ELISA test described in step 5 above, wherein C3b was replaced by 20-100 ng each of C3c (100), C3d (20), C3$\alpha$ (20) and C3$\beta$ (20).

As a result it was found that the binding curves of the eluate E and the original antibodies O exhibited almost identical binding curves for the fragment C3c and the $\alpha$-chain, the binding increasing considerably with increasing IgG concentration. In contrast thereto the curves for the binding to C3d and the C3$\beta$ chain were found to differ. In both cases the preparation O presented steep binding curves without a horizontal level, while the curves of E levelled off at half (C3d) and 2/3 (C3$\beta$-chain), respectively, of the maximal binding obtained by O. The binding curves of the eluate C from the control rabbit-IgG column were found to have identical height and slopes as those of O for all the C3 fragments.

The results obtained thus indicate that E and O exhibited a similar binding to C3b (step 4 above), C3c and the $\alpha$-chain, while the binding of E was limited to restricted range of epitopes in C3d and the $\beta$-chain compared to O. The binding curves for the eluate C from the control IgG column had the same height and slope as those of the original antibodies O for the different C3 fragments, which again demonstrates that the anti-C3 antibodies occuring in the eluate C were non-specifically bound to the column.

7. **Comparison of the binding specificity of the preparations E and O in immunoblotting**

By means of elastase two factor C3 fragments, C3c and C3d, were generated, which were reduced and subjected to sodium dodecyl sulphate (SDS) polyacrylamide gel electrophoresis (PAGE) according to the metod described by Laemmli, U.K., and Favre, M.J., J. Mol. Biol. 80:575 (1973). Samples of the reduced fragments containing 5% by volume of mercaptoethanol were incubated for 5 minutes at 100°C before the electrophoresis.

The binding specificities of the eluate E obtained in step 4 above and of the non-affinity purified antibody solution O with regard to the separated C3 fragments obtained in the electrophoresis were compared by means of a modification of the immunoblotting technique described by Eggertsen, G., et al, Immunol. 131:1920-1923 (1983). The two antibody solutions E and O were in this case diluted to give an antigen excess. The bound antibodies were visualized by incubation of the nitro cellulose membrane with swine anti-rabbit IgG conjugated with horseradish peroxidase (HRP) (DAKO Immunoglobulins A/S, Denmark) for one hour at room temperature followed by rinsing with PBS and incubation in "HRP Colour Development Reagent" (containing 4-chloro-1-naphtol; Bio Rad Lab., USA) according to the manufacturer's recommendations. The nitro cellulose paper was then photographed and the film was developed. A transparent positive copy was then run in a Quick Scan Jr. gel scanner (Helena Laboratories, USA) for a semiquantitative intensity measurement. The area under the obtained graph was used as a measure of the intensity of the individual bands. The results are listed in the following Table I.

## TABLE I

| Experiment | Specificity of the original antibodies | Ratio of staining intensities (E/O) | | |
|---|---|---|---|---|
| | | β-chain | 40 kd | C3d |
| 1 | native C3 | 0.29 | 0.81 | 0.51 |
| 2 | C3α-β | 0.52 | 1.26 | 0.56 |
| 3 | C3α-β | 0.65 | 0.95 | — |
| 4 | native C3 | 0.72 | 1.32 | 0.84 |
| mean ± SEM[x] | | 0.55 ± 0.09 | 1.09 ± 0.12 | 0.63 ± 0.10 |

[x] The ratio of the 40 kd fragment is significantly higher than the ratios of the β-chain and the C3d fragment ($p < 0.05$).

It was found that both the antibody preparations E and O were bound to the β-chain, to the 40 kd fragment of C3c and to C3d. The 20 kd fragment of C3c was not visible with either of the antibody preparations. The binding of E and O to the β-chain and C3d were similar, while the binding of E to the 40 kd fragment of C3 was stronger than for O (Table I, Experiment 1). Identical experiments performed with the use of eluates of anti-native C3 and anti-C3α,β from a second anti-factor H column (section 4 above) showed similar binding ratios (Table I, Experiment 2-4). The results demonstrate that an enrichment of antibodies against C3c is obtained, which indicates that C3c is a major binding site for factor H.

Hereinafter some experiments will be described which demonstrate that the affinity purified antibodies from step 4 above (eluate E) may serve as a substitute for factor H as a co-factor to factor I.

8. Inhibition of the activity of anti-C3 antibodies by factor H

100 ng of factor C3 were preadsorbed to each well of a microtitre plate at +4°C. 50 μl of serially diluted factor H from step 1 above were then added, followed by 50 μl of constant doses of anti-C3 antibodies from the antibody solutions O and E obtained in step 4 above, and incubation was allowed for one hour at room temperature. The anti-C3 doses of each antibody solution was adjusted to give the same amount of rabbit IgG bound to the preadsorbed C3 in the uninhibited controls (i.e. without added factor H). Swine anti-rabbit IgG-HRP conjugate followed by colour reagent were then added and an optical measurement was performed in the same way as in the ELISA test described in step 5 above.

As a result it was found that factor H inhibited both the affinity purified (E) and the original anti-C3 antibodies. However, inhibition of the affinity purified antibody preparation only required a 25th part of factor H compared with that required to inhibit the binding of the original antibodies. This indicates that the anti-C3 antibodies eluted from the anti-factor H column have similar binding properties as factor H.

9. Test of affinity purified anti-C3 (E) as co-factor to factor I

5 μg of C3b were incubated with 1 μg of factor I and three-fold serial dilutions of the eluate E obtained in step 4 above for 3 hours at 37°C. A corresponding incubation of C3b was performed in the presence of 1.6 μg of factor H and 1 μg of factor I. Finally a corresponding control experiment was performed in the absence of factor I. Then sodium dodecyl sulphate polyacrylamide gel electrophoresis (SDS-PAGE) was performed on all the test preparations. Both the incubation with E and with factor H were found to give rise to three new fragments with the approximate molecular weights of 65, 45 and 42 kd, respectively. With increasing concentrations of E-antibodies the fragmentation seemed, however, to reach an optimum without a complete fragmentation of the α'-chain. For the control experiment in the absence of factor I only trace amounts of the above mentioned fragments were visible.

To obtain a quantitative measure of the α'-chain split another experiment was performed, where 1.5 μg of $^{125}$I-labelled C3b were incubated with 1 μg of factor I and serially diluted eluate E and original antibody solution O, respectively, from step 4 above for 180 minutes at 37°C followed by SDS-PAGE.

The labelling of the C3b molecule with $^{125}I$ was performed with Na $^{125}I$ (Amersham, England) by a lactoperoxidase technique to obtain a specific activity of 40,000 cpm/µg protein, employing "Enzymobeads" (Bio Rad Lab., USA) according to the manufacturer's recommendations. The bands containing the α'-chains were cut out from the electrophoresis gel and quantitated by mesurement of the $^{125}I$-activity. It was found that the original antibody solution O had no significant effect on the degradation of C3b, even when the concentration was increased 10 times, whereas the eluate E clearly promoted chain split. However, more than 20% fragmentation of the α'-chain could not be obtained. The necessary dose of factor H to obtain a 20% degradation was 100 ng, and a total split was obtained in the presence of 1,600 ng.

The results indicate that the affinity purified antibodies of the eluate E function as a co-factor to factor I and thus that the antibodies which are specific to the binding sites against factor H were successfully selected from these polyclonal antisera. The observed differences in properties in relation to factor H may i.a. be assigned to the fact that among the eluted antibodies E there are synergistic antibodies which bind to the active functional binding site but also antibodies (probably in great excess) which bind to other parts of the binding region, thereby inhibiting the action of the synergistic antibodies. The antibodies per se also have a higher affinity to C3b than factor H, and once the antibody has bound to the C3b molecule it therefore easily catalyzes the fragmentation of the α'-chain, but due to the high affinity it will not with a sufficient rate be reacted in binding reactions with other C3b molecules. Furthermore, when in fluid-phase the divalent polyclonal antibodies might be considered to form antigen-antibody complexes which enclose the antigen and thereby hinder it from participating in any reaction.

## EXAMPLE II

This example illustrates the application of the invention to the production of antibodies specifically directed against rat IgE receptor.

1. Production of antisera against RBL-1 and IR2, respectively

Antisera were produced, on one hand, against a rat basophil leukemia cell-line (RBL-1 - described by Eccleston, E. et al, Nature 244:73-76 (1973)) (190 ml) and, on the other hand, rat IgE (IR2 - described by Bazin H. et al, Immunology 26:713-723 (1974)) (225 ml) by immunization of rabbits. These two antisera were purified by first performing a buffer change on a G25 column (Pharmacia AB, Sweden) (column size K50/100, total volume $V_t$ = 1000 ml) to 75 mM tris-HCl, pH 8.0. The buffer change was followed by ion exchange chromatography on DEAE-Sepharose® (Pharmacia AB, Sweden) (K50/30, $V_t$ = 500

ml). The IgG containing fractions were combined and chromatographed on lysine-Sepharose® (Pharmacia AB, Sweden) (K16/20, $V_t$ = 20 ml) to eliminate the major part of the proteolytic activity.

2. <u>Coupling of the anti-IR2 material to solid phase</u>

290 mg of the anti-IR2 material obtained above was immobilized on CNBr-activated Sepharose® CL4B (Pharmacia AB, Sweden) in the way recommended by the manufacturer. The anti-IR2 Sepharose® material obtained was packed in a column (K16/20).

3. <u>Affinity chromatogrophy of anti-RBL-1</u>

575 mg of the anti-RBL-1 material obtained in step 1 above was divided into three equal pools which were each treated in identical manner. All the pools were first passed through a column to obtain normal rabbit IgG coupled to CNBr-activitated Sepharose® CL4B (218 mg, K16/20, $V_t$ = 17 ml). This step was performed to eliminate background activity, i.e. such antibodies that were expected to bind to determinants common to all rabbit IgG molecules. The three background purified pools were then chromatographed on the anti-IR2-Sepharose® column prepared in step 2 above, the column first being run a cycle without sample. Each cycle comprised the following buffer steps: PBS, pH 7.2, 150 ml; 0.5 M NaCl, 100 ml; 0.1 M glycine-HCl, 0.5 M NaCl, pH = 3.0, 50 ml; 0.5 M NaCl, 50 ml; 0.1 M glycine-HCl, 0.5 M NaCl, pH = 3.0, 50 ml; PBS, pH 7.2, 100 ml. In total 1.1 mg of antibody in 50 ml of buffer were obtained from the first acid elution of the three chromatographies. pH was then adjusted to neutral with 0.5 M NaOH.

According to the inventive concept the antibody obtained should in its idiotype resemble parts of the receptor interacting surface of the rat IgE molecule and be expected to bind to the RBL-1 receptor therefor. That this is indeed the case will be demonstrated below.

4. <u>Demonstration of specificity to rat IgE receptor by immunoblotting</u>

a) <u>Preparation of different cell lysates</u>

<u>RBL-1 cells:</u> $1.41 \times 10^8$ cells were repeatedly washed in PBS, pH 7.2, and dissolved in 850 µl of PBS. 100 µl of lysing buffer (0.5% NP 40 (Sigma, USA), 35 mM PMSF (phenylmethyl sulfonyl fluoride - Sigma, USA), 35 mM iodine acetamide in PBS, pH = 7.2) were added to the cells, and the mixture was then subjected to end-over-end rotation at room temperature for 20 minutes. The lysate was then centrifuged in an Eppendorf centrifuge at 10,000 rpm for 5 minutes. The clear supernatant was transferred to a new tube and prepared for polyacrylamide gel electrophoresis (PAGE).

<u>Rat mast cells:</u> $5-6 \times 10^6$ cells were washed and dissolved in 450 µl of

16

PBS and 50 µl of lysing buffer were added. The cells were then treated as described above.

Rat peritoneal cells (a mixture of lymphocytes, monocytes, macrophages and eosinophiles): an unknown number of cells were treated in identical manner as described above for the mast cells.

Rat thymus cells: an unknown number of cells were dissolved in 1200 µl of PBS and added to 120 µl of lysing buffer. The cells were then treated as described above for the other cell types.

b) Electrophoresis

The four cell lysates obtained above were each treated separately for electrophoresis as follows: SDS mix (18% SDS in 0.24 M tris, 0.12 M Na-acetate, 10.8 mM EDTA, pH = 7.4) was added to the samples in the ratio of 1:5. The samples were then boiled in a water bath for 2 minutes, after which they were mixed with pyronine marker (50% glycerol, 500 µg of pyronine Y (Sigma, USA) per ml) in the ratio of 1:5. A 12.6% polyacrylamide gel was then cast, with which electrophoresis of all the above mentioned cell lysates in triplicate was performed (10 µl of RBL-1 lysate, 50 µl of mast and peritoneal cell lysates, respectively, 10 µl of thymus cell lysate) at 100 volts for 5-6 hours.

c) Immunoblotting

After completed electrophoresis the gel obtained together with a nitro cellulose membrane were transferred to a blotting assembly, and the proteins from the gel were elctrophoretically transferred to the nitro cellulose membrane for 2 hours at 100 volts. Remaining free binding surfaces on the nitrocellulose membrane were inactivated in PBS-1% Tween® 20 buffer at room temperature overnight, and the membrane was then cut into three identical membrane pieces. The first membrane piece was protein coloured with 0.1% amidoblack in 45% methanol, 10% acetic acid, while the other two membrane pieces were subjected to the immunocolouring described below.

d) Immunocolouring

One of the two membrane pieces obtained in the immunoblotting process c) above which had not been protein coloured was incubated for 60 minutes with the affinity purified anti-RBL-1 antibody obtained under section 3 above diluted to 5 µg/ml in PBS-0.05% Tween® 20. The other membrane piece was incubated in the same way with normal rabbit IgG to be used as a control. The membranes were then thoroughly washed in PBS-1% Tween® 20 and incubated for 60 minutes with sheep fab-anti rabbit-Fc$\gamma$ alkaline phosphatase (MIAB, Sweden), diluted to 1:100. The two membrane pieces were then washed again as before and incubated with enzyme substrate obtained by mixing the following solutions A and B in equal volumes; solution A: Naphtol AS-MX (Sigma, USA), 0.4 mg/ml in distilled $H_2O$; solution B: Fast Red TR Salt (Sigma, USA), 6.0 µg/ml in 0.2 M tris-HCl, pH = 8.2. After about 15 minutes red bands occurred where antibody had bound to protein in the membrane.

e) <u>Results</u>

The protein colouring performed above exhibited a plurality of very closely spaced distinct bands for all the lysates except the mast cell lysate, for which only a few week bands were noticable. In contrast the immunocolouring in the corresponding case produced only two distinct bands for the RBL-1 lysate, and these bands plus a third adjacent band for the peritoneal cell lysate. The mast cell lysate only gave the third band, and the thymus cell lysate exhibited no bands at all. Similar immunocolouring with original (not affinity purified) anti-RBL-1 antiserum produced a great number of bands against the RBL-1 lysate. The normal rabbit IgG control was completely blank for all the lysates. The molecular weight of these three bands detected in the immunocolouring was 60-70 K, which is well in conformity with the size of the rat IgE receptor. The anti-RBL-1 preparation purified on the anti-IR2 column is thus specifically directed against the IgE receptors of the cells.

Alternatively, in the corresponding way as described above, an affinity purified anti-rat IgE preparation (which is to resemble the RBL-1 receptor) may, of course, be produced instead by coupling the anti-RBL-1 material obtained in step 1 to the column material of step 2.

The invention is, of course, not restricted to the above described embodiments and examples, but may be varied and modified in many ways within the scope of the accompanying claims.

18

0241444

CLAIMS

1. An immunological method comprising the production of antibodies capable of reacting with a specific part of a contact surface of either of two macromolecules interacting to form a complex, characterized by

a) separately producing polyclonal antisera containing antibodies against the respective macromolecules,

b) contacting the immunoglobulin fractions of said antisera with each other, such that complementary antibodies may react to form antibody complexes,

c) separating the pairs of complexed antibodies obtained from antibodies which have not reacted with each other, and

d) dissociating said antibody complexes and separately recovering at least one antibody component of said antibody complexes.

2. A method according to claim 1, characterized in that in step b) the immunoglobulin fraction of one antiserum is immobilized to a solid phase.

3. A method according to claim 2, characterized in that said solid phase immobilized immunoglobulin fraction is provided in a column through which the other antiserum is passed.

4. A method according to claim 1, 2 or 3, characterized by, before or after step b), subjecting one or both of said antisera to an adsorptive purification step for removing non-specifically reacting immunoglobulins.

5. A method according to any one of claims 1-4, characterized in that said two macromolecules do not constitute an antigen-antibody pair.

6. A method according to any one of claims 1-5, characterized in that by means of the antibody or antibodies obtained in step d) an antigen taking part in said macromolecular interaction, or a fragment thereof, is identified and recovered from a mixture containing the corresponding macromolecule or fragment thereof, respectively.

19 0241444

7. A method according to claim 6,
characterized in that the antigen or antigen fragment obtained is used for screening monoclonal antibodies.

8. A method according to claim 6,
characterized in that the antigen or antigen fragment obtained is used for producing vaccines or antisera for passive immune protection.

9. A method according to any one of claims 1-5,
characterized in that the antibody or antibodies obtained are used as a substitute antigen.

10. A method according to claim 9,
characterized in that the substitute antigen is used in a diagnostic test.

11. A method according to claim 9,
characterized in that the substitute antigen is used for screening monoclonal antibodies.

12. A method according to any one of the preceding claims,
characterized in that one of said macromolecules is at least a part of a receptor on a cell surface.

FIG. 1A.

FIG. 1B

FIG. 1C

0241444

FIG. 2

0241444

European Patent Office

**EUROPEAN SEARCH REPORT**

. Application number

87850117.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A2-141 783 (THE WISTAR INSTITUTE) . <br> * page 6, last paragraph - page 7, first paragraph * <br> ---- | 1-3, 8, 9 | G 01 N 33/531 <br> A 61 K 39/395 <br> C 07 K 15/00 <br> G 01 N 33/577 <br> G 01 N 33/566 |
| A | SCIENCE, vol. 228, no. 4696, April 12, 1985, pages 162-165, Washington (US), J.L. MARX: "Making Antibodies without the Antigens" <br> * page 164, right column * <br> ---- | 1-3, 8, 12 | |
| A | BIO/TECHNOLOGY, vol. 2, November 1984, pages 923 and 927, ZOLER M.L.: "Anti--idiotypic antibodies: A safer way to immunize against AIDS ?" <br> * the whole article * <br> ---- | 1-3, 8 | |
| Y | NATURE, vol. 305, September 1, 1983, pages 56-57, London (GB), W.L. CLEVELAND: "Monoclonal antibodies to the acetylcholine receptor by a normaly functioning auto-anti-idiotypic mechanism" <br> * page 57, left column * <br> ---- . | 1-12 | **TECHNICAL FIELDS SEARCHED (Int. Cl.4)** <br><br> A 61 K <br> C 07 K <br> G 01 N |
| E | WO-A1-87/01458 (THE TRUSTEES OF COLUMBIA UNIVERSITY IN THE CITY OF NEW YORK) <br> * page 2, line 6 - page 4; page 17, lines 9-25 * <br> ---- | 1-3, 6 | |

The present search report has been drawn up for all claims

| Place of search | Date of completion of the search | Examiner |
|---|---|---|
| STOCKHOLM | 09-07-1987 | GUSTAFSSON C-O |